# EUROPEAN PATENT SPECIFICATION

(11) **EP 3 355 707 B1**
(45) Date of publication and mention of the grant of the patent: **08.05.2024**
(21) Application number: 16852774.5
(22) Date of filing: 30.09.2016
(51) Int. Cl.: A23G 4/00, A23G 4/08, A23G 4/06

(54) **HIGHLY HOMOGENOUS HYDROPHILIC GUM BASES**
HOCH HOMOGENE HYDROPHILE KAUGUMMIGRUNDMASSEN
BASES DE GOMMES HYDROPHILES HAUTEMENT HOMOGÈNES

(30) Priority: 30.09.2015 US 201562235431 P
(43) Date of publication of application: 08.08.2018
(73) Proprietor: Wm. Wrigley Jr. Company, Chicago, IL 60642 (US)
(72) Inventor: LIU, Jingping, Chicago, IL 60642 (US)
(74) Representative: Haseltine Lake Kempner LLP
(86) International application number: PCT/US2016/054975
(87) International publication number: WO 2017/059347

(56) References cited:
- EP-A2- 0 066 864
- WO-A1-95/03710
- WO-A1-2010/127250
- WO-A2-00/08944
- WO-A2-2014/039755
- US-A- 4 297 290
- US-A- 4 452 820
- US-A- 4 452 820
- US-A- 4 931 293
- US-A- 5 064 658
- US-A- 6 153 231
- US-A1- 2015 230 495
- BURGOS ET AL.: 'Synthesis and Characterization of Lactic Acid Oligomers: Evaluation of Performance as Poly(Lactic Acid) Plasticizers' JOURNAL OF POLYMERS AND THE ENVIRONMENT vol. 22, no. 2, pages 227 - 235, XP055386035
- RIKEN: 'Kinds of Food Emulsifiers', [Online] 20 August 2015, page 1, 3, XP055386040 Retrieved from the Internet: <URL:http://web.archive.org/web/20150820075 736/ http://www.rikenvitamin.com/foodingredients /emuls mer/kind./html pg> [retrieved on 2016-11-29]

## Description

### FIELD

The presently disclosed subject matter relates to chewing gum base compositions comprising polymer material and at least one miscible plasticizer.

### BACKGROUND

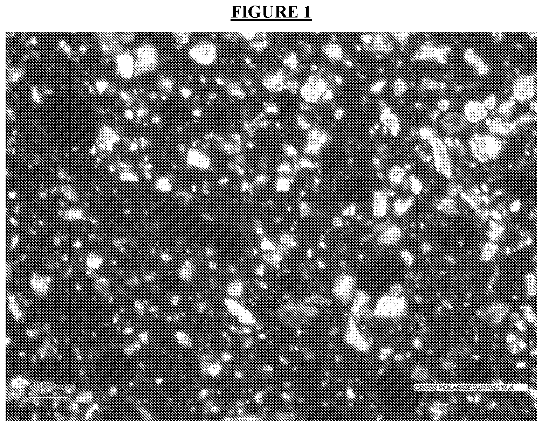

Commercial chewing gum is known to contain soluble and insoluble portions. The insoluble portion contains mainly gum bases and hydrophobic gum components, for example, masticatory polymers, tackifiers, plasticizers, lipids, fillers, emulsifiers, flavor residues and other additives. The insoluble portion of conventional gum is heterogeneous due to the immiscibility of different ingredients. As shown in Figure 1, hydrophobic elastomers form a continuous phase with hydrophobic tackifiers. High melting point fats and waxes disperse as crystal domains within the gum base. Polyvinyl acetate (PVAc) has relatively high hydrophilicity due to acetate groups present along the polymer backbone. Accordingly, it is immiscible with other hydrophobic elastomers used in the chewing gums, such as butyl rubber, polyisobutylene and styrene-butyldiene rubber. As a result, there is a phase separation between PVAc and the other polymeric phases. Depending on the formulation, PVAc forms either a dispersive amorphous domain (the black spots as shown in Figure 1) or a separate continuous phase within the gum base matrix.

Since the interactions between these different phases are usually weak in gum, the cohesion of the conventional chewing gum matrix is reduced further due to the heterogeneity. When adhesion on a surface is higher than cohesion, the gum matrix tends to leave behind residue during removal. There remains a need in the art to increase gum cohesion to improve removability. The presently disclosed subject matter addresses this need through various means.

The presently disclosed subject matter provides for a highly homogenous gum matrix, such as rubber-free PVAc gum base, which can increase the cohesion of gum for improved removability. Further, PVAc has a lower holding ability for minty flavor chemicals than common base elastomers, such as butyl rubber and polyisobutylene. The presently disclosed subject matter provides for rubber-free PVAc gum base that can improve flavor release. Additionally, conventional gums tend to be visually opaque. This is because the individual components typically have different refractive indices and are in different microdomains, which can strongly scatter light, causing bulk samples to have an opaque appearance. The presently disclosed subject matter provides for a highly homogenous gum matrix, such as rubber-free PVAc gum base, which can increase matrix clarity and transparency.

Furthermore, in order to become pliable, PVAc requires a certain level of hydration or plasticization. However, there are few effective, low off-taste and safe miscible plasticizers to increase pliability for PVAc found in chewing gum industrials. Therefore, there remains a need in the art to form pliable PVAc-based gum bases.

US4452820 discloses a gum base which is formed essentially of a properly plasticized elastomer.

WO 2014/039755 discloses chewing gum bases comprising a block polymer comprising at least four blocks and having an Order-Disorder Transition Temperature (T_{ODT}) between 20 °C and 250 °C.

WO 95/03710 relates to wax-free chewing gum and bubble gum bases and methods of making the same, and discloses gum bases that are substantially free of wax but have been modified to increase the glass transition of the base.

WO 2010/127250 discloses a chewing gum composition including: a gum base and a gingivitis-inhibiting amount of hyaluronic acid of a physiologically acceptable salt thereof.

US5064658 pertains to sweetened chewing gum compositions which comprise (A) a gum base; (B) a bulking agent; (C) a flavoring agent; and (D) an encapsulated synergistic sweetening agent composition.

US4931293 is concerned with chewing gum compositions having prolonged acid release during mastication comprising a gum base and a stable food delivery system capable of effecting a controlled release of the acid.

WO 00/08944 is directed to non-stick chewing gum compositions containing plasticized proteinaceous materials which are derived from the combination or blend of at least one protein and at least one plasticizer. EP0066864 discloses a chewing gum base comprising 25% PVA.

### SUMMARY OF THE INVENTION

The presently disclosed subject matter is directed to a rubber free chewing gum base comprising polyvinyl acetate and 5 to 50% by weight of the gum base of at least one miscible plasticizer selected from the group consisting of hydroxyl carboxylic acid ester from aliphatic alcohols with chain length of C₂-C₁₂, and hydroxyl mono- or di- or tri- carboxylic acids with chain length of C₂-C₈, and/or sorbitan esters of fatty acid with chain length of C₁₀-C₁₆, and/or poly(lactic acid) oligomers (mw<2000), and/or propylene glycol esters of fatty acid with chain length of C₈-C₁₂ and/or propylene glycol esters of benzonic acid, and combinations thereof, or the plasticizer is selected from the group consisting of: aliphatic dicarboxylic acid esters from aliphatic alcohols with chain length of C₂-C₁₂, dicarboxylic acids with chain length of C₂-C₈, and combinations thereof

In other embodiments, the plasticizer is selected from the group consisting of: hydroxyl carboxylic acid ester from aliphatic alcohols with chain length of C₂-C₈, and hydroxyl mono- or di- or tri- carboxylic acids with chain length of C₂-C₄, butyl lactate, triethyl citrates, dibutyl malate, and combinations thereof.

In other embodiments, the plasticizer is selected from the group consisting of: aliphatic dicarboxylic acid esters from aliphatic alcohols with chain length of C₂-C₈, and dicarboxylic acids with chain length of C₂-C₄, dibutyl sebacate, dioctyl sebacate, dibutyladipate, bis(2-ethylhexyl)adipate, dibutyl succinate, dioctyl succinate, and combinations thereof.

In other embodiments, the emulsifier is selected from the group consisting of: sorbitan esters of fatty acid with chain length of C₁₀-C₁₆, and combinations thereof.

In other embodiments, the plasticizer is selected from the group consisting of: poly(lactic acid) oligomers (mw<2000), and combinations thereof.

In other embodiments, the plasticizer is selected from the group consisting of: propylene glycol esters of fatty acid with chain length of C₈-C₁₂ or benzonic acid, propylene glycol dicaprylocaprate, propylene glycol mono/dilaurate, polypropylene glycol dibenzoate, di-/polypropylene glycol dibenzonate, and combinations thereof.

In other embodiments, the plasticizer is selected from the group consisting of: unsaturated fatty acids with chain length of C₁₆-C₂₀, and combinations thereof.

In other embodiments, the plasticizer is selected from the group consisting of: unsaturated fatty acids with chain length of C₁₈, oleic acid, and combinations thereof.

In other embodiments, the gum base further comprises an emulsifier. In certain embodiments, the emulsifier is selected from the group consisting of: monoglycerides of fatty acids with chain length of C₆-C₁₂, and combinations thereof.

In other embodiments, the emulsifier is selected from the group consisting of: monoglycerides of fatty acids with chain length of C₈-C₁₀, capric acid mono-/diglycerides and caprylic mono-/diglycerides, and combinations thereof.

In other embodiments, the emulsifier is selected from the group consisting of: sorbitan esters of fatty acid with chain length of C₁₀-C₁₆, sorbitan monolaurate, sorbitan monopalmitate, and combinations thereof.

In other embodiments, the emulsifier is selected from the group consisting of: sorbitan esters of fatty acid with chain length of C₁₂, sorbitan monolaurate, sorbitan monopalmitate, propylene glycol monolaurate, and combinations thereof.

The presently disclosed subject matter is further directed to a chewing gum formulation comprising the chewing gum base described herein.

The foregoing has outlined broadly the features and technical advantages of the present application in order that the detailed description that follows can be better understood. Additional features and advantages of the application will be described hereinafter which form the subject of the claims of the application. It should be appreciated by those skilled in the art that the conception and specific embodiment disclosed can be readily utilized as a basis for modifying or designing other structures for carrying out the same purposes of the present application. It should also be realized by those skilled in the art that such equivalent constructions do not depart from the scope of the application as set forth in the appended claims. The novel features which are believed to be characteristic of the application, both as to its organization and method of operation, together with further objects and advantages will be better understood from the following description.

### BRIEF DESCRIPTION OF THE DRAWINGS

**Figure 1** provides a cross polarized optical microscopy graph of the insoluble portion of typical conventional gum.

### DETAILED DESCRIPTION

As noted above, to date, there remains a need in the art for effective, low off-taste and safe miscible plasticizers to increase pliability for polyvinyl acetate (PVAc) gum bases. The presently disclosed subject matter addresses this need as discussed below.

### 1. Definitions

The terms used in this specification generally have their ordinary meanings in the art, within the context of this disclosed subject matter and in the specific context where each term is used. Certain terms are discussed below, or elsewhere in the specification, to provide additional guidance to the practitioner in describing the compositions and methods of the disclosed subject matter and how to make and use them.

As used herein, the use of the word "a" or "an" when used in conjunction with the term "comprising" in the claims and/or the specification can mean "one," but it is also consistent with the meaning of "one or more," "at least one," and "one or more than one." Still further, the terms "having," "including," "containing" and "comprising" are interchangeable and one of skill in the art is cognizant that these terms are open ended terms.

The term "about" or "approximately" means within an acceptable error range for the particular value as determined by one of ordinary skill in the art, which will depend in part on how the value is measured or determined, *i*.*e*., the limitations of the measurement system. For example, "about" can mean within 3 or more than 3 standard deviations, per the practice in the art. Alternatively, "about" can mean a range of up to 20%, preferably up to 10%, more preferably up to 5%, and more preferably still up to 1% of a given value.

As used herein, the term "chewing gum" refers to a flavored substance intended for chewing. The term as used herein also includes bubble gum and confectionery products containing chewing gum. In certain embodiments, chewing gum forms include, but are not limited to, tablets, sticks, solid balls, hollow balls, cut and wrap, and pellets or pillows. Unless otherwise specified, all percentages used herein are weight percentages. As used herein, chewing gum contains a water insoluble base portion and a water-soluble bulk portion.

As used herein, the term "gum cud" refers to the remaining portion of a chewing gum formulation after mastication.

### 2. Gum Base Compositions

The presently disclosed subject matter relates to compositions of chewing gum, and more specifically to chewing gum bases, which can include a polymer material and at least one miscible plasticizer. The addition of at least one miscible plasticizer can improve the pliability and removability of chewing gum and/or chewing gum cud.

In general, a chewing gum composition comprises (i) a water-soluble bulk portion, (ii) water insoluble flavoring agents, and (iii) a water insoluble chewable gum base portion. The water-soluble bulk portion dissipates with a portion of the water insoluble flavoring agents over a period of time during chewing. However, the water insoluble gum base portion is retained in the mouth throughout the chew, forming what is known as gum cud. The produced gum cud is an adhesive substance that is difficult to remove once attached to dry surfaces (e.g., sidewalks and the like).

### 2.1. Insoluble Gum Bases

Water insoluble gum bases can contain any combination of elastomers, vinyl polymers, elastomer plasticizers, fillers, softeners, waxes and other optional ingredients such as colorants and antioxidants. Many ingredients are immiscible on a molecular level and the result is a highly heterogeneous system with different dispersive and continuous phases.

In general, the continuous phase is composed of a hydrophobic elastomer/tackifier network. This network can contain different dispersive phases with different domain sizes. The sizes of the dispersive phases vary from several microns to 100 or more microns after aging. Because the interactions between the many different phases are usually poor and the interfacial energy between the incompatible phases tends to rise, structural defects are produced.

The glass transition temperature of the dispersive phase is usually higher than the glass transition temperature of the continuous elastomer phase. At ambient temperature, the dispersive phases behave more like a glassy solid, and the continuous phase is in a rubbery state. The glassy domains can be more rigid than the continuous elastomer phase and as a result, decreases the removability of the overall gum base composition.

The subject matter disclosed herein provides for gum bases that have less removing residue than the conventional chewing gum. An improved or increased homogeneity of gum base can improve the gum cud removability as well as optical clarity. Gum bases with a hydrophilic continuous phase can also alter the releasing pattern of the water soluble phase during mastication.

### 2.1.1 Homogenous hydrophilic gum base

The presently disclosed subject matter provides for hydrophilic gum bases that are highly homogenous and clear. In specific embodiments, the gum bases are formulated from at least one masticatory polymer ingredient and at least one softening agent.

In certain embodiments, the masticatory polymeric ingredients include but are not limited to polyvinyl acetate (PVAc), vinyl acetate-vinyl laurate copolymer, poly-dl-lactide, glycolic acid-lactide copolymer, polyhydroxyalkanoates or mixtures thereof. In certain embodiments, the average molecular weight of the masticatory polymeric ingredient is in the range of from about 10⁴ to about 10⁶ g/mole, or from about 10⁴ to about 10⁵ g/mole. In a specific embodiment, the masticatory polymeric ingredient is PVAc.

In specific embodiments, the gum base includes from about 50 to about 95 weight percent polymeric materials, or from about 60 to about 80 weight percent polymeric materials.

The softening agent can include at least one plasticizer and/or at least one emulsifier. In certain embodiments, the plasticizers and emulsifiers are miscible with the polymeric ingredient on a molecular level.

In certain embodiments, a plasticizer is selected from the group consisting of: hydroxyl carboxylic acid ester from aliphatic alcohols with a chain length of C₂-C₁₂, or C₂-C₈, and hydroxyl mono- or di- or tri- carboxylic acids with a chain length of C₂-C₈, or C₂-C₄, which includes but is not limited to butyl lactate, triethyl citrates, dibutyl malate, and combinations thereof. In other embodiments, the plasticizer is selected from the group consisting of: aliphatic dicarboxylic acid esters from aliphatic alcohols with a chain length of C₂-C₁₂ or C₂-C₈, and dicarboxylic acids with chain length of C₂-C₈, or C₂-C₄, which includes but is not limited dibutyl sebacate, dioctyl sebacate, dibutyladipate, bis(2-ethylhexyl)adipate, dibutyl succinate, dioctyl succinate, and combinations thereof. In other embodiments, the plasticizer is selected from the group consisting of: sorbitan ester of fatty acid with a chain length of C₁₀-C₁₆ or C₁₂, which includes but is not limited to sorbitan monolaurate, sorbitan monopalmitate, and combinations thereof.

In certain embodiments, the plasticizer is selected from the group consisting of: propylene glycol esters of fatty acid with a chain length of C₈-C₁₂ or benzonic acid, which includes but is not limited to propylene glycol dicaprylocaprate, propylene glycol mono/dilaurate, polypropylene glycol dibenzoate, di-/polypropylene glycol dibenzonate, and combinations thereof. In other embodiments, the plasticizer is a poly(lactic acid) oligomer (mw<2000).

In other embodiments, the plasticizer is selected from the group consisting of: triethyl citrate, butyl lactate, 2-ethylhexyl-s-lactate, capric acid monoglycerides, capric acid diglycerides, caprylic glycerides, capric glycerides, polypropylene glycol dibenzoate, dibutyl sebacate, and combinations thereof.

In other embodiments, the plasticizer is dibutyl sebacate. In other embodiments, the plasticizer is bis(2-ethylhexyl)adipate. In other embodiments, the plasticizer is 2-ethylhexyl-s-lactate.

The gum base includes from about 5% to about 50% w/w plasticizers, or from about 10% to about 30% w/w plasticizer. In certain embodiments, the gum base includes about 20% w/w, about 24% w/w, or about 28% w/w plasticizers.

Emulsifiers can accelerate the hydration rate of PVAc in saliva or lower its glassy transition temperature, turning it from glassy material to soft rubbery chewy substance.

In certain embodiments, an emulsifier is selected from the group consisting of: acetylated monoglycerides of fatty acid with chain length of C₁₆-C₁₈, which includes but is not limited to acetylated monoglyceride of stearic acid, and combinations thereof. In other embodiments, the emulsifier is selected from the group consisting of: monoglycerides of fatty acids with a chain length of C₆-C₁₂ or C₈-C₁₀, which includes but is not limited to capric acid mono-/diglycerides and caprylic mono-/diglycerides, and combinations thereof. In other embodiments, the emulsifier is selected from the group consisting of: sorbitan ester of fatty acid with a chain length of C₁₀-C₁₆ or C₁₂, which includes but is not limited to sorbitan monolaurate and sorbitan monopalmitate, propylene glycol monolaurate, and combinations thereof.

In specific embodiments, the gum base includes from about 1% to about 15% w/w emulsifiers, or from about 2% to about 10% w/w emulsifiers. In certain embodiments, the gum base includes about 4% w/w emulsifiers.

The gum base of the presently disclosed subject matter can also include one or more colorants, whiteners, and/or antioxidants. In certain embodiments, the colors and whiteners can include, but are not limited to, FD&C-type dyes and lakes, fruit and vegetable extracts, titanium dioxide or mixtures thereof. In certain embodiments, the antioxidants can include, but are not limited to, beta-carotenes, acidulants (*e*.*g*., Vitamin C), alpha-tocopherol (vitamin E), beta, gamma and delta tocopherols, propyl gallate, butylated hydroxyanisole (BHA), butylated hydroxytoluene (BHT) and tertiary butyl hydroquinone (TBHQ). In specific embodiments, the gum base includes optional minor amounts (about one percent or less) of miscellaneous ingredients such as colorants, antioxidants, etc.

In certain embodiments, the gum bases are free of hydrophobic elastomers, hydrophobic resins, minerals and triglycerides.

### 3. Chewing Gum

The presently disclosed subject matter includes chewing gum formulations comprising the above disclosed gum base compositions. Methods of making the chewing gum formulations are also contemplated.

### 3.1 Formulations

In certain embodiments, the gum base compositions disclosed above can be incorporated into chewing gum formulations. In certain embodiments, the chewing gum formulation can include one of the disclosed water-insoluble gum base compositions and a water-soluble bulking agent portion. The water soluble portion of the chewing gum typically includes a bulking agent (also called bulk sweeteners) together with minor amounts of secondary components such as flavoring agents (including sensates such as physiological cooling agents, warming agents and tingling agents), high-intensity sweeteners, colorants, humectants, gum emulsifiers, acidulants, fillers, and binders. Typically, the water-soluble portion, sensates, and flavors dissipate during chewing and the gum base is retained in the mouth throughout the chew.

In certain embodiments, bulk sweeteners can include both sugars and sugar alcohols. Bulk sweeteners can constitute about 5% to about 95% by weight of the chewing gum, more typically, about 20% to about 80% by weight, and more commonly, about 30% to about 60% by weight of the gum. Sugar sweeteners generally include saccharide-containing components commonly known in the chewing gum art, including but not limited to, sucrose, dextrose, maltose, dextrin, dried invert sugar, fructose, galactose, corn syrup solids, and the like, alone or in combination. Sugarless sweeteners include, but are not limited to, sugar alcohols such as sorbitol, mannitol, xylitol, hydrogenated starch hydrolysates, maltitol, erythritol, isomalt and the like, alone or in combination.

In certain embodiments, a variety of flavoring agents can also be used, if desired. Flavoring agents can include essential oils, synthetic flavors or mixtures thereof including, but not limited to, oils derived from plants and fruits such as citrus oils, fruit essences, peppermint oil, spearmint oil, other mint oils, clove oil, oil of wintergreen, anise and the like. Artificial flavoring agents and components can also be used. Natural and artificial flavoring agents can be combined in any sensorially acceptable fashion. Included in the general category of flavors are sensates, chemicals which impart physiological sensations in the mouth such as cooling agents, warming agents and tingling agents. Examples of cooling agents include menthol, WS-23, WS-3, WS-5, isopulegol, esters of menthol such as menthyl succinate, menthyl lactate and menthyl glutarate, among others. Warming and tingling agents include capsaicin, piperine, jambu and spilanthol.

In certain embodiments, the flavor agent can be used in amounts of about 0.1 to about 15 weight percent, and preferably, about 0.2% to about 5% by weight of the gum formulation. In certain embodiments, the flavoring agent is present in an amount within the range of from about 0.1% to about 10.0% by weight or from about 0.5% to about 3.0% by weight of the gum formulation. In certain embodiments, the flavoring agent is present in an amount of about 0.9% by weight of the gum formulation. In the case of fruit flavored gums, up to about 3% by weight of an acid such as citric acid, malic acid or adipic acid can be added for tartness.

In some embodiments, the chewing gum ingredients will include one or more high intensity sweeteners. As used herein, the term "high intensity sweetener" refers to any substance that is at least twenty times sweeter than sucrose. Such sweeteners include saccharin, cyclamate, aspartame, alitame, neotame, other peptide-based sweeteners, sucralose, acesulfame K, stevia (including purified extracts such as rebaudioside A), glycyrrhizin, neohesperidin dihydrochalcone and mixtures thereof. In some embodiments, at least a portion of the high intensity sweetener will be encapsulated. Such encapsulations can be produced by granulation, agglomeration, extrusion and grinding, spray drying, fluid bed encapsulation or any other known means. In certain embodiments, suitable sugar alcohols include sorbitol, mannitol, xylitol, hydrogenated starch hydrolysates, maltitol, and the like, as well as combinations thereof. In certain embodiments, the sugarless gum comprises a combination of a high-potency sweetener with a sugar alcohol, *e*.*g*., aspartame and sorbitol. Usage levels will depend on the potency of the sweetener, degree and effectiveness of the encapsulation (if any) as well as the sensory profile desired for the product. Generally, the sweetener can be used in a range of from about 0.005% to about 5% w/w. The sweetener can be used at levels as low as about 0.005% w/w or as low as about 0.05% w/w or as low as about 0.2% w/w. The sweetener can be used at levels as high as about 5% w/w or about 3% w/w or about 2% w/w in the chewing gum composition. In certain embodiments, the high intensity sweetener can be present at a level of from about 0.1% to about 1.0% of the chewing gum ingredients.

In certain embodiments, fillers include, but are not limited to, magnesium and calcium carbonate, ground limestone and silicate types such as magnesium and aluminum silicate, clay, alumina, talc as well as titanium oxide, mono-, di- and tricalcium phosphate, cellulose polymers such as ethyl, methyl and wood or mixtures thereof, and combinations thereof.

In certain embodiments, humectants are added to the water soluble portion of chewing gum in order to optimize the chewability and mouth feel of the gum. In certain embodiments, humectants, also known in the art as plasticizers or plasticizing agents, can constitute between about 0.5% to about 15.0% by weight of the chewing gum formulation. Softeners include, but are not limited to, glycerin, lecithin, and combinations thereof. Further, aqueous sweetener solutions such as those containing sorbitol, hydrogenated starch hydrolysates, corn syrup, and combinations thereof, can be used as softeners and binding agents in chewing gum formulations. In certain embodiments, the chewing gum formulation contains about 1% w/w glycerin. In certain embodiments, the chewing gum formulation contains about 1.4 %w/w glycerin by weight. In certain embodiments, the gum base compositions as disclosed herein, can be incorporated into a sugarless chewing gum formulation. In certain embodiments, sugarless sweeteners can include components with sweetening characteristics but that are devoid of the commonly known sugars. In certain embodiments, examples include, but are not limited to high-potency sweeteners and/or sugar alcohols.

Combinations of sugar and/or sugarless sweeteners can be used in chewing gum. Additionally, the softener can also provide additional sweetness such as with aqueous sugar or alditol solutions. In certain embodiments, the water-soluble sweetener portion is a mixture of sugar at about 50% of the final chewing gum, dextrose monohydrate at about 10%, and/or corn syrup at about 17%. In certain embodiments, the formulation comprises about 60% sucrose and/or about 17% corn syrup. In certain embodiments the formulation comprises about 58.7% sucrose and/or about 17% corn syrup.

If a low calorie gum is desired, a low calorie bulking agent can be used. Examples of low calorie bulking agents include: polydextrose; oligofructose (Raftilose); inulin (Raftilin); fructooligosaccharides (NutraFlora); palatinose oligosaccharide; guar gum hydrolysate (BeneFiber); or indigestible dextrin (Fibersol). However, other low calorie bulking agents can be used.

In certain embodiments, pharmaceutical agents can be added to the chewing gum formulation.

In certain embodiments, the insoluble gum base composition constitutes between about 5% to about 95% by weight of the chewing gum formulation. In certain embodiments, the insoluble gum base composition comprises between about 10% and about 50%, or about 20% to about 35%, percent by weight of the chewing gum formulation.

### 3.2 Methods of Making

The presently disclosed subject matter includes methods of making a chewing gum formulation comprising the gum base compositions discussed above.

In certain embodiments, chewing gum is manufactured by sequentially adding the various chewing gum ingredients to a commercially available mixer known in the art. In certain embodiments, after the ingredients have been thoroughly mixed, the gum mass can be discharged from the mixer and shaped into the desired form such as by rolling into sheets and cutting into sticks, extruding into chunks or casting into pellets or deposit into different molds.

In certain embodiments, the ingredients are mixed by first melting the gum base and adding it to the running mixer. In certain embodiments, the base can be melted in the mixer itself. In certain embodiments, color or emulsifiers can be added at this time. In certain embodiments, a softener, for example, glycerin, can be added at this time along with syrup and a portion of bulking agent. In certain embodiments, further portions of the bulking agent can then be added to the mixer. In certain embodiments a flavoring agent is added with the final portion of the bulking agent. In certain embodiments, a twice coated sweetener can be added after the final portion of bulking agent and flavor have been added.

In some embodiments a coating can be applied to the extruded chewing gum. In certain embodiments, this is accomplished by pan coating the expanded chewing gum piece. In a pan coating process, centers (*e*.*g*., expanded chewing gum pieces) are tumbled in a pan while a coating syrup, typically a sugar or sugar alcohol solution, is applied, for instance by spraying or ladling. Between applications of the coating syrup, the pellets are dried, preferably by passing a current of heated and/or dried air over or through the pellet bed. Numerous layers of coating are built up, often alternating with applications of powdered coating material or an inert filler to accelerate the build-up of the coating. A final layer of a polishing compound, for example carnauba wax, can be applied. In addition to sugar (sucrose), preferred coating materials include maltitol, isomalt, xylitol, sorbitol and erythritol, although others can be used as well. In addition to the coating material, the coating syrup can include film forming agents such as Gum Arabic, high intensity sweeteners, flavors and colors.

### EXAMPLES

The presently disclosed subject matter will be better understood by reference to the following Examples, which are provided as exemplary of the disclosed subject matter, and not by way of limitation.

### Example 1. Miscibility test of polyvinyl acetate with different softeners

This Example provides an evaluation of miscibility of PVAc with various softeners. A summary of the results is provided in Table 1.

Binary polyvinylacetate/plasticizer thin films were prepared by polymer solution casting. The PVAc/plasticizer ratio was 70/30 by weight. Solvents were selected from tetrahydrofuran (THF) and chloroform. Solute content was from about 5% to about 10% by weight. The solution was casted within an aluminum dish with smooth surface. The solvent was evaporated under a hood overnight at ambient temperature and then in a vacuum oven at around 55°C, under maximum vacuum for 24 hours. The film was evaluated based on visual appearance and a finger tack test. Clarity and wholesomeness of the film, without leaching liquid, were characteristics considered for miscibility. The deformability of the film was considered for plasticization strength. The plasticizers which formed easy-to-deform, clear films without liquid leaching, were considered excellent.

**Table 1. Miscibility of various plasticizers with PVAc.**

| **Plasticizer** | **Chemical name** | **Vendor** | **Film Appearance** | **Miscibility** |
|---|---|---|---|---|
| SAIB | Sucrose acetate isobutyrate | Eastman Chemical | Homogeneous, hard | OK |
| TEC (Morflex) | Triethyl Citrate | Morflex, Inc. | Homogeneous, soft | Excellent |
| PLA oligomer | Poly(lactic acid), MW<2000 | LADCO Lab | Homogeneous, soft | Good |
| Butyl lactate (Purac) | Butyl lactate | Purac Biochem | Clear, very soft | Excellent |
| Lactem P22 | Lactic acid ester of monoglyceride | Danisco | Homogeneous, hard | OK |
| Acetem 95CO | Acetylated monoglycerides | Danisco | Homogeneous, soft | Excellent |
| PURASOL EHL | 2-ethylhexyl-s-lactate | PURAC | Clear, very soft | Excellent |
| LAUROGLYCOL | Propylene glycol laurate | Gattefosse | Translucent, soft | Good |
| LAUROGLYCOL 90 | Propylene glycol monolaurate | Gattefosse | Translucent, soft | Good |
| Aldo MO | Glyceryl monooleate | Lonza Inc | Clear, soft | Good |
| CLA-DG | Propylene glycol linoleate | Stepan Co | Cloudy, flexible | OK |
| IMWITOR 312 | Monoglyceride of lauric acid | SASOL | Opaque, flexible | Poor |
| IMWITOR 988 | Capric acid mono-/diglycerides / caprylic/capric glycerides | SASOL | Clear, soft | Excellent |
| Beecap 5 | Triglyceride of capric acid | Stepan Co. | Oily, flexible | OK |
| GLYCOMUL TS | Sobitan tristearate | Lonza Inc. | Opaque, firm | Poor |
| PanadanSDK | DATEM (liquid) | Danisco | Homogeneous, hard | Poor |
| GLYCOMUL L | sorbitan monolaurate | LONZA | Clear, soft | Good |
| GLYCOLMUL S | Sorbitan monostearate | LONAZ | Opaque, firm | Poor |
| LABRAFAC PG | Propylene glycol dicaprylocaprate | Gattefosse | Translucent, soft Taste OK | Good |
| OLEIC ACID | Oleic acid | Spectrum | Clear, soft | Good |
| DOA | Bis(2-ethylhexyl)adipate | Eastman | Clear, flexible | Good |
| CitremLR-10 | Citric acid ester of monoglyceride | Danisco | Firm | Poor |
| RIKEMAL B-50 | succinylated monoglyceride | RIKEVITA( Malaysia) SDN.BHD | Opaque, firm | Poor |
| PGPR90 | Polyglycerol polyricinoleate | Danisco | Firm | Poor |
| Uniplex400 | Polypropylene glycol dibenzoate | Unitex Chemical co. | Soft, clear | Excellent |
| Morflex 190 | Butyl phthalyl butyl glycolate | Morflex, Inc. | Soft, clear, taste OK | Excellent |
| Dibutyl malate | di-n-butyl malate | Morflex, Inc. | Soft, clear, bitter | Good |
| Citroflex 8 | Tri(2-ethylhexyl) citrate | Morflex, Inc. | Clear, tough, taste OK | Good |
| DBS | Dibutyl sebacate | Morflex, Inc. | Clear, soft, taste OK | Excellent |
| S/B Flk #795 | Hydrogenated soya oil | Humko | Opaque, crystalline, hard | Poor |
| CSO Flk #787 | Hydrogenated cotton seed oil | Humko | Opaque, crystalline, hard | Poor |
| Sp. Dreyfus Wax Blend | Paraffin & microcrystalline wax | Petrolite | Crystalline, firm | Poor |
| PS 8814 #1590 | Partially hydrogenated soya oil | Humko | Crystalline, firm | Poor |
| Super G 932 | Glycerol monostearate (soya oil) | Humko | Crystalline, firm | Poor |

### Example 2. Gum base containing dibutyl sebacate as main plasticizer

This Example provides a gum base formulation comprising dibutyl sebacate as the plasticizer.

In a gum base mixer (Plastograph from Brabender Corp., Rochelle Park, N.J.) set at 100° C, 73 g polyvinyl acetate (Wacker Chemie GmbH, Germany) was heated and stirred with a sigma blade stirrer. When the material in the mixer had achieved a substantially single homogeneous plastic mass, 20 g butyl dibutyl sebacate (Morflex, Inc., Greensboro, NC) was added in increments of 1/3. The mixture was stirred for about five minutes after each incremental addition. Then, 3.5 g glycerol triacetate (Eastman Chemical Company, Kingsport, TN) was added and the mixture was stirred for about 5 minutes. This was followed by step-wise additions of 3.5 g acetylated monoglyceride (Danisco Cultor USA, Inc.) in increments of 1/3 portions. The mixture was stirred for about five minutes after each addition.

The resulting base was clear and had comparable consistency with conventional gum base.

### Example 3. Gum base containing bis(2-ethylhexyl)adipate and oleic acid as plasticizers and glyceryl monooleate as emulsifier

This Example provides a gum base formulation comprising bis(2-ethylhexyl)adipate and oleic acid as the plasticizers.

In a gum base mixer (Plastograph from Brabender Corp., Rochelle Park, N.J.) set at 100° C, 68 g polyvinyl acetate (Wacker Chemie GmbH, Germany) was heated and stirred with a sigma blade stirrer. When the material in the mixer had achieved a substantially single homogeneous plastic mass, 24 g bis(2-ethylhexyl)adipate (Eastman Chemical Company, Kingsport, TN) was added in increments of 1/3 each, followed by stirring for about five minutes after each incremental addition. Then, 4 g oleic acid (Spectrum Chemical Company, New Brunswick, NJ) was added and stirred for about 5 minutes, followed by step-wise additions of 4 g glyceryl monooleate (Lonza Inc., Fair Lawn, NJ) in increments of 1/3 portions of the overall amount, followed by stirring for about five minutes for each portion. Then, 0.1 g antioxidant BHT was added with 2 minutes of stirring.

The resulting base was clear and had comparable consistency with conventional gum base.

### Example 4. Gum base containing 2-ethylhexyl-s-lactate and butyl lactate as plasticizers and sorbitan monolaurate as emulsifier

This Example provides a gum base formulation comprising 2-ethylhexyl-s-lactate and butyl lactate as plasticizers.

In a gum base mixer (Plastograph from Brabender Corp., Rochelle Park, N.J.) set at 100° C, 68 g polyvinyl acetate (Wacker Chemie GmbH, Germany) was heated and stirred with a sigma blade stirrer. When the material in the mixer had achieved a substantially single homogeneous plastic mass, 24 g 2-ethylhexyl-s-lactate (Purac America Inc., Lincolnshire, IL) was added in increments of 1/3 each followed by stirring about five minutes after each incremental addition. Then, 4 g butyl lactate (Purac America Inc., Lincolnshire, IL) was added and stirred for about 5 minutes followed by step-wise additions of 4 g sorbitan monolaurate, (Lonza Inc., Fair Lawn, NJ) in increments of 1/3. This was followed by stirring for about five minutes for each portion. Then, 0.1 g antioxidant BHT was added with 2 minutes of stirring.

The resulting base was clear and had comparable consistency with conventional gum base.

### Example 5. Gum base containing tri(2-ethylhexyl) citrate and triethyl citrate as plasticizers

Example 2 was repeated using tri(2-ethylhexyl) citrate and triethyl citrate instead of dibutyl sebacate and glycerol triacetate.

In a gum base mixer (Plastograph from Brabender Corp., Rochelle Park, N.J.) set at 100° C, 73 g polyvinyl acetate (Wacker Chemie GmbH, Germany) was heated and stirred with a sigma blade stirrer. When the material in the mixer had achieved a substantially single homogeneous plastic mass, 20 g tri(2-ethylhexyl) citrate was added in increments of 1/3. The mixture was stirred for about five minutes after each incremental addition. Then, 3.5 g triethyl citrate was added and the mixture was stirred for about 5 minutes. This was followed by step-wise additions of 3.5 g acetylated monoglyceride (Danisco Cultor USA, Inc.) in 1/3 portions. The mixture was stirred for about five minutes after each addition.

The resulting base was clear and had comparable consistency with conventional gum base.

### Example 6. Gum base containing poly(lactic acid) oligomer and di-n-butyl malate as plasticizers

Example 2 was repeated using poly(lactic acid) oligomer (mw<2000) and di-n-butyl malate instead of dibutyl sebacate and glycerol triacetate

In a gum base mixer (Plastograph from Brabender Corp., Rochelle Park, N.J.) set at 100° C, 73 g polyvinyl acetate (Wacker Chemie GmbH, Germany) was heated and stirred with a sigma blade stirrer. When the material in the mixer had achieved a substantially single homogeneous plastic mass, 20 g poly(latic acid) oligomer was added in increments of 1/3. The mixture was stirred for about five minutes after each incremental addition. Then, 3.5 g di-n-butyl malate was added and the mixture was stirred for about 5 minutes. This was followed by step-wise additions of 3.5 g acetylated monoglyceride (Danisco Cultor USA, Inc.) in 1/3 portions. The mixture was stirred for about five minutes after each addition.

The resulting base was clear and had comparable consistency with conventional gum base.

### Example 7. Gum base containing propylene glycol laurate as main plasticizer and propylene glycol monolaurate as emulsifier

Example 2 was repeated using propylene glycol laurate and propylene glycol monolaurate instead of dibutyl sebacate and acetylated monoglyceride, respectively.

In a gum base mixer (Plastograph from Brabender Corp., Rochelle Park, N.J.) set at 100° C, 73 g polyvinyl acetate (Wacker Chemie GmbH, Germany) was heated and stirred with a sigma blade stirrer. When the material in the mixer had achieved a substantially single homogeneous plastic mass, 20 g propylene glycol laurate was added in increments of 1/3. The mixture was stirred for about five minutes after each incremental addition. Then, 3.5 g glycerol triacetate (Eastman Chemical Company, Kingsport, TN) was added and the mixture was stirred for about 5 minutes. This was followed by step-wise additions of 3.5 g propylene glycol monolaurate in 1/3 portions. The mixture was stirred for about five minutes after each addition.

The resulting base was clear and had comparable consistency with conventional gum base.

### Example 8. Gum base containing propylene glycol dicaprylocaprate

Example 2 was repeated using propylene glycol dicaprylocaprate instead of dibutyl sebacate.

In a gum base mixer (Plastograph from Brabender Corp., Rochelle Park, N.J.) set at 100° C, 73 g polyvinyl acetate (Wacker Chemie GmbH, Germany) was heated and stirred with a sigma blade stirrer. When the material in the mixer had achieved a substantially single homogeneous plastic mass, 20 g butyl propylene glycol dicaprylocaprate was added in increments of 1/3. The mixture was stirred for about five minutes after each incremental addition. Then, 3.5 g glycerol triacetate (Eastman Chemical Company, Kingsport, TN) was added and the mixture was stirred for about 5 minutes. This was followed by step-wise additions of 3.5 g acetylated monoglyceride (Danisco Cultor USA, Inc.) in 1/3 portions. The mixture was stirred for about five minutes after each addition.

The resulting base was clear and had comparable consistency with conventional gum base.

### Example 9. Gum base containing polypropylene glycol dibenzoate

Example 2 was repeated using polypropylene glycol dibenzoate instead of dibutyl sebacate.

In a gum base mixer (Plastograph from Brabender Corp., Rochelle Park, N.J.) set at 100° C, 73 g polyvinyl acetate (Wacker Chemie GmbH, Germany) was heated and stirred with a sigma blade stirrer. When the material in the mixer had achieved a substantially single homogeneous plastic mass, 20 g polypropylene glycol dibenzoate was added in increments of 1/3. The mixture was stirred for about five minutes after each incremental addition. Then, 3.5 g glycerol triacetate (Eastman Chemical Company, Kingsport, TN) was added and the mixture was stirred for about 5 minutes. This was followed by step-wise additions of 3.5 g acetylated monoglyceride (Danisco Cultor USA, Inc.) in 1/3 portions. The mixture was stirred for about five minutes after each addition.

The resulting base was clear and had comparable consistency with conventional gum base.

### Example 10. Gum base containing dipropylene glycol dibenzoate

Example 2 was repeated using dipolypropylene glycol dibenzoate instead of dibutyl sebacate.

In a gum base mixer (Plastograph from Brabender Corp., Rochelle Park, N.J.) set at 100° C, 73 g polyvinyl acetate (Wacker Chemie GmbH, Germany) was heated and stirred with a sigma blade stirrer. When the material in the mixer had achieved a substantially single homogeneous plastic mass, 20 g dipolypropylene glycol dibenzoate was added in increments of 1/3. The mixture was stirred for about five minutes after each incremental addition. Then, 3.5 g glycerol triacetate (Eastman Chemical Company, Kingsport, TN) was added and the mixture was stirred for about 5 minutes. This was followed by step-wise additions of 3.5 g acetylated monoglyceride (Danisco Cultor USA, Inc.) in 1/3 portions. The mixture was stirred for about five minutes after each addition.

The resulting base was clear and had comparable consistency with conventional gum base.

### Example 11. Preparation of chewing gum formulations

This Example provides a chewing gum formulation, as described in Table 2, comprising a base of the previous Examples.

In a gum base mixer (Plastograph from Brabender Corp., Rochelle Park, N.J.) set at 65° C, 21 g the gum base prepared in Example 2 and 1/6 sugar were added and stirred with a sigma blade stirrer until the material reached a fine particulate consistency. Corn syrup and glycerin were added after base smearing, and mixed for 1 minute and 30 seconds. 1/3 of the sugar was added in portions over a 1 minute duration and mixed for three minutes. Flavor was added over 30 second durations and mixed for 1 minute and 30 seconds; 1/2 of the sugar was added in 1.5 minute duration and mixed for 4 minutes. The total mixing time is 18 minutes.

**Table 2. Gum formulation.**

| %(wt.) | GUM |
|---|---|
| Example 2 | 21 |
| Sucrose | 59.7 |
| Corn syrup | 17 |
| Glycerin | 1.4 |
| Flavor | 0.9 |

### Example 12. Removability test of gum cuds

This Example provides the removability of the chewing gum formulations prepared in Example 11.

Gum samples of Example 11 and a control were water washed. Each chewed gum cud was taken out of water. One set of samples (3 pieces) was placed on a concrete surface with a release paper on the top of the gum cud. Gum cuds were then sandwiched between 2 concrete blocks and pressed by weight (>120lbs). The samples between the concrete blocks were heated at temperature of 90° F for 24 hours. After removing the concrete block and the release paper, the samples were aged for 3 days at ambient temperature. Another set of samples (3 pieces) was placed on the sidewalk pavement with a release paper on the top of the gum cud. Gum cuds were then pressed by weight (>120lbs). After removed the release paper, the samples were aged for 4 months at ambient environment (May-August in New Jersey).

A handhold scrapper with force gauge (EXTECH Instruments 475044) was used to scrape the gum cud samples from the concrete block. The results of the removability test are summarized in Table 3.

**Table 3. Removability results.**

| | Scraper force (lb) | Residual (%) |
|---|---|---|
| Example 11 | 23 | 5 |
| Control | 23 | 89 |
| Example 11 (aged 4 months) | 17 | 15 |
| Control (aged 4 months) | 15 | 100 |

The results indicate that while the control gum cuds could barely be removed, the gum cuds of the presently disclosed subject matter are highly removable.

Although the presently disclosed subject matter and its advantages have been described in detail, it should be understood that various changes, substitutions and alterations can be made herein without departing from the scope of the disclosed subject matter as defined by the appended claims. Moreover, the scope of the present application is not intended to be limited to the particular embodiments of the process, machine, manufacture, composition of matter, means, methods and steps described in the specification. As one of ordinary skill in the art will readily appreciate from the disclosure of the presently disclosed subject matter, processes, machines, manufacture, compositions of matter, means, methods, or steps, presently existing or later to be developed that perform substantially the same function or achieve substantially the same result as the corresponding embodiments described herein can be utilized according to the presently disclosed subject matter. Accordingly, the appended claims are intended to include within their scope such processes, machines, manufacture, compositions of matter, means, methods, or steps.

## Claims

1. A chewing gum base comprising polyvinyl acetate and 5 to 50% by weight of the gum base of at least one miscible plasticizer selected from the group consisting of hydroxyl carboxylic acid esters from aliphatic alcohols with chain length of C₂-C₁₂, and hydroxyl mono- or di- or tri- carboxylic acids with chain length of C₂-C₈, and/or sorbitan esters of fatty acid with chain length of C₁₀-C₁₆, and/or poly(lactic acid) oligomers (mw<2000), and/or propylene glycol esters of fatty acid with chain length of C₈-C₁₂ and/or propylene glycol esters of benzonic acid, and combinations thereof, or aliphatic dicarboxylic acid esters from aliphatic alcohols with a chain length of C₂-C₁₂ and dicarboxylic acids with a chain length of C₂-C₈, wherein the chewing gum base is rubber free.

2. The chewing gum base of claim 1, wherein the plasticizer is selected from the group consisting of: hydroxyl carboxylic acid esters from aliphatic alcohols with chain length of C₂-C₈, and hydroxyl mono- or di- or tri- carboxylic acids with chain length of C₂-C₄, butyl lactate, triethyl citrates, dibutyl malate, and combinations thereof.

3. The chewing gum base of claim 1, wherein the plasticizer is selected from the group consisting of: aliphatic dicarboxylic acid esters from aliphatic alcohols with chain length of C₂-C₈, dicarboxylic acids with chain length of C₂-C₄, dibutyl sebacate, dioctyl sebacate, dibutyladipate, bis(2-ethylhexyl)adipate, dibutyl succinate, dioctyl succinate, and combinations thereof.

4. The chewing gum base of claim 1, wherein the plasticizer is selected from the group consisting of: sorbitan esters of fatty acid with chain length of C₁₀-C₁₆, and combinations thereof.

5. The chewing gum base of claim 1, wherein the plasticizer is selected from the group consisting of: poly(lactic acid) oligomers (mw<2000), and combinations thereof.

6. The chewing gum base of claim 1, wherein the plasticizer is selected from the group consisting of: propylene glycol esters of fatty acid with chain length of C₈-C₁₂, propylene glycol esters of benzonic acid, propylene glycol dicaprylocaprate, propylene glycol mono/dilaurate, polypropylene glycol dibenzoate, di-/polypropylene glycol dibenzonate, and combinations thereof.

7. The chewing gum base of claim 1, wherein the plasticizer is selected from the group consisting of: unsaturated fatty acids with chain length of C₁₆-C₂₀, and combinations thereof

8. The chewing gum base of claim 7, wherein the plasticizer is selected from the group consisting of: unsaturated fatty acids with chain length of C₁₈, oleic acid, and combinations thereof.

9. The chewing gum base of claim 1, wherein the gum base further comprises an emulsifier.

10. The chewing gum base of claim 9, wherein the emulsifier is selected from the group consisting of: monoglycerides of fatty acids with chain length of C₆-C₁₂, and combinations thereof.

11. The chewing gum base of claim 10, wherein the emulsifier is selected from the group consisting of: monoglycerides of fatty acids with chain length of C₈-C₁₀, capric acid mono-/diglycerides and caprylic mono-/diglycerides, and combinations thereof.

12. The chewing gum base of claim 9, wherein the emulsifier is selected from the group consisting of: sorbitan esters of fatty acid with chain length of C₁₀-C₁₆, sorbitan monolaurate, sorbitan monopalmitate, and combinations thereof.

13. The chewing gum base of claim 9, wherein the emulsifier is selected from the group consisting of: sorbitan esters of fatty acid with chain length of C₁₂, sorbitan monolaurate, sorbitan monopalmitate, propylene glycol monolaurate, and combinations thereof.

14. The chewing gum base of claim 1, wherein the gum base comprises from 10 to 30% by weight of the gum base of plasticizer

15. A chewing gum formulation comprising the chewing gum base of any one of the preceding claims

## Patentansprüche

1. Kaugummigrundmasse, umfassend Polyvinylacetat und 5 bis 50 Gew.-% der Gummigrundmasse mindestens eines aus der aus Hydroxylcarbonsäureestern von aliphatischen Alkoholen mit einer Kettenlänge von C₂-C₁₂ und Hydroxylmono- oder -di- oder -tricarbonsäuren mit einer Kettenlänge von C₂-C₈ und/oder Sorbitanestern von Fettsäuren mit einer Kettenlänge von C₁₀-C₁₆ und/oder Polymilchsäureoligomeren (MW <2000) und/oder Propylenglykolestern von Fettsäure mit einer Kettenlänge von C₈-C₁₂ und/oder Propylenglykolestern von Benzonsäure und Kombinationen davon oder aliphatischen Dicarbonsäureestern von aliphatischen Alkoholen mit einer Kettenlänge von C₂-C₁₂ und Dicarbonsäuren mit einer Kettenlänge von C₂-C₈ bestehenden Gruppe ausgewählten mischbaren Weichmachers, wobei die Kaugummigrundmasse gummifrei ist.

2. Kaugummigrundmasse nach Anspruch 1, wobei der Weichmacher aus der aus Hydroxylcarbonsäureestern von aliphatischen Alkoholen mit einer Kettenlänge von C₂-C₈ und Hydroxylmono- oder -di- oder -tricarbonsäuren mit einer Kettenlänge von C₂-C₄, Butyllactat, Triethylcitraten, Dibutylmalat und Kombinationen davon bestehenden Gruppe ausgewählt ist.

3. Kaugummigrundmasse nach Anspruch 1, wobei der Weichmacher aus der aus aliphatischen Dicarbonsäureestern von aliphatischen Alkoholen mit einer Kettenlänge von C₂-C₈, Dicarbonsäuren mit einer Kettenlänge von C₂-C₄, Dibutylsebacat, Dioctylsebacat, Dibutyladipat, Bis(2-ethylhexyl)adipat, Dibutylsuccinat, Dioctylsuccinat und Kombinationen davon bestehenden Gruppe ausgewählt ist.

4. Kaugummigrundmasse nach Anspruch 1, wobei der Weichmacher aus der aus Sorbitanestern von Fettsäuren mit einer Kettenlänge von C₁₀-C₁₆ und Kombinationen davon bestehenden Gruppe ausgewählt ist.

5. Kaugummigrundmasse nach Anspruch 1, wobei der Weichmacher aus der aus Polymilchsäureoligomeren (MW <2000) und Kombinationen davon bestehenden Gruppe ausgewählt ist.

6. Kaugummigrundmasse nach Anspruch 1, wobei der Weichmacher aus der aus Propylenglykolestern von Fettsäuren mit einer Kettenlänge von C₈-C₁₂, Propylenglykolestern von Benzonsäure, Propylenglykoldicaprylocaprat, Propylenglykolmono/dilaurat, Polypropylenglykoldibenzoat, Di-/Polypropylenglykoldibenzonat und Kombinationen davon bestehenden Gruppe ausgewählt ist.

7. Kaugummigrundmasse nach Anspruch 1, wobei der Weichmacher aus der aus ungesättigten Fettsäuren mit einer Kettenlänge von C₁₆-C₂₀ und Kombinationen davon bestehenden Gruppe ausgewählt ist.

8. Kaugummigrundmasse nach Anspruch 7, wobei der Weichmacher aus der aus ungesättigten Fettsäuren mit einer Kettenlänge von C₁₈, Ölsäure und Kombinationen davon bestehenden Gruppe ausgewählt ist.

9. Kaugummigrundmasse nach Anspruch 1, wobei die Gummigrundmasse weiterhin einen Emulgator umfasst.

10. Kaugummigrundmasse nach Anspruch 9, wobei der Emulgator aus der aus Monoglyceriden von Fettsäuren mit einer Kettenlänge von C₆-C₁₂ und Kombinationen davon bestehenden Gruppe ausgewählt ist.

11. Kaugummigrundmasse nach Anspruch 10, wobei der Emulgator aus der aus Monoglyceriden von Fettsäuren mit einer Kettenlänge von C₈-C₁₀, Caprinsäuremono-/diglyceriden und Caprylsäuremono-/diglyceriden und Kombinationen davon bestehenden Gruppe ausgewählt ist.

12. Kaugummigrundmasse nach Anspruch 9, wobei der Emulgator aus der aus Monoglyceriden von Fettsäuren mit einer Kettenlänge von Sorbitanestern von Fettsäuren mit einer Kettenlänge von C₁₀-C₁₆, Sorbitanmonolaurat, Sorbitanmonopalmitat und Kombinationen davon bestehenden Gruppe ausgewählt ist.

13. Kaugummigrundmasse nach Anspruch 9, wobei der Emulgator aus der aus Sorbitanestern von Fettsäuren mit einer Kettenlänge von C₁₂, Sorbitanmonolaurat, Sorbitanmonopalmitat, Propylenglykolmonolaurat und Kombinationen davon bestehenden Gruppe ausgewählt ist.

14. Kaugummigrundmasse nach Anspruch 1, wobei die Gummigrundmasse 10 bis 30 Gew.-% der Gummigrundmasse an Weichmacher umfasst.

15. Kaugummiformulierung, umfassend die Kaugummigrundmasse nach einem der vorhergehenden Ansprüche.

## Revendications

1. Base de chewing-gum comprenant un poly(acétate de vinyle) et 5 à 50 % en poids de la base de gomme d'au moins un plastifiant miscible sélectionné dans le groupe constitué par des esters d'acides hydroxycarboxyliques d'alcools aliphatiques dotés d'une longueur de chaîne de C₂-C₁₂, et d'acides hydroxymonocarboxyliques ou hydroxydicarboxyliques ou hydroxytricarboxyliques dotés d'une longueur de chaîne de C₂-C₈ et/ou des esters de sorbitane d'acide gras doté d'une longueur de chaîne de C₁₀-C₁₆, et/ou d'oligomères de poly(acide lactique) (mw < 2 000), et/ou des esters de propylèneglycol d'acide gras doté d'une longueur de chaîne de C₈-C₁₂ et/ou des esters de propylèneglycol d'acide benzonique et des combinaisons correspondantes, ou des esters d'acides dicarboxyliques aliphatiques d'alcools aliphatiques dotés d'une longueur de chaîne C₂-C₁₂ et d'acides dicarboxyliques dotés de longueur de chaînes de C₂-C₈, la base de chewing-gum étant exempte de caoutchouc.

2. Base de chewing-gum selon la revendication 1, dans laquelle le plastifiant est sélectionné dans le groupe constitué par : esters d'acides hydroxycarboxyliques d'alcools aliphatiques dotés d'une longueur de chaîne de C₂-C₈, et acides hydroxymonocarboxyliques ou hydroxydicarboxyliques ou hydroxytricarboxyliques dotés d'une longueur de chaîne de C₂-C₄, lactate de butyle, citrates de triéthyle, malate de dibutyle et leurs combinaisons.

3. Base de chewing-gum selon la revendication 1, dans laquelle le plastifiant est sélectionné dans le groupe constitué par : esters d'acides dicarboxyliques aliphatiques d'alcools aliphatiques dotés d'une longueur de chaîne de C₂-C₈, acides dicarboxyliques dotés d'une longueur de chaîne de C₂-C₄, sébaçate de dibutyle, sébaçate de dioctyle, adipate de dibutyle, adipate de bis(2-éthylhexyle), succinate de dibutyle, succinate de dioctyle et leurs combinaisons.

4. Base de chewing-gum selon la revendication 1, dans laquelle le plastifiant est sélectionné dans le groupe constitué par : esters de sorbitane d'acides gras dotés d'une longueur de chaîne de C₁₀-C₁₆, et leurs combinaisons.

5. Base de chewing-gum selon la revendication 1, dans laquelle le plastifiant est sélectionné dans le groupe constitué par : oligomères (mw < 2 000) de poly(acide lactique), et leurs combinaisons.

6. Base de chewing-gum selon la revendication 1, dans laquelle le plastifiant est sélectionné dans le groupe constitué par : esters de propylène glycol d'acides gras dotés d'une longueur de chaîne de C₈-C₁₂, esters de propylène glycol d'acide benzonique, dicaprylocaprate de propylène glycol, mono/dilaurate de propylène glycol, dibenzoate de polypropylène glycol, dibenzonate de di-/polypropylène glycol, et leurs combinaisons.

7. Base de chewing-gum selon la revendication 1, dans laquelle le plastifiant est sélectionné dans le groupe constitué par : acides gras insaturés dotés d'une longueur de chaîne de C₁₆-C₂₀, et leurs combinaisons.

8. Base de chewing-gum selon la revendication 7, dans laquelle le plastifiant est sélectionné dans le groupe constitué par : acides gras insaturés dotés d'une longueur de chaîne de C₁₈, acide oléique, et leurs combinaisons.

9. Base de chewing-gum selon la revendication 1, dans laquelle la base de gomme comprend en outre un émulsifiant.

10. Base de chewing-gum selon la revendication 9, dans laquelle l'émulsifiant est sélectionné dans le groupe constitué par : monoglycérides d'acides gras dotés d'une longueur de chaîne de C₆-C₁₂, et leurs combinaisons.

11. Base de chewing-gum selon la revendication 10, dans laquelle l'émulsifiant est sélectionné dans le groupe constitué par : monoglycérides d'acides gras dotés d'une longueur de chaîne de C₈-C₁₀, mono-/diglycérides d'acide caprique et mono-/diglycérides capryliques, et leurs combinaisons.

12. Base de chewing-gum selon la revendication 9, dans laquelle l'émulsifiant est sélectionné dans le groupe constitué par : esters de sorbitane d'acides gras dotés d'une longueur de chaîne de C₁₀-C₁₆, monolaurate de sorbitane, monopalmitate de sorbitane et leurs combinaisons.

13. Base de chewing-gum selon la revendication 9, dans laquelle l'émulsifiant est sélectionné dans le groupe constitué par : esters de sorbitane d'acides gras dotés d'une longueur de chaîne de C₁₂, monolaurate de sorbitane, monopalmitate de sorbitane, monolaurate de propylène glycol et leurs combinaisons.

14. Base de chewing-gum selon la revendication 1, dans laquelle la base de gomme comprend de 10 à 30 % en poids de la base de gomme de plastifiant.

15. Formulation de chewing-gum comprenant la base de chewing-gum selon l'une quelconque des revendications précédentes.
